Europäisches Patentamt

European Patent Office

Office européen des brevets

(11) Publication number: **0 644 201 A1**

(12) **EUROPEAN PATENT APPLICATION**

(21) Application number: **94202409.2**

(22) Date of filing: **21.09.91**

(51) Int. Cl.6: **C07K 14/16**, C12N 15/49, A61K 39/39, A61K 39/21

This application was filed on 23 - 08 - 1994 as a divisional application to the application mentioned under INID code 60.

(30) Priority: **28.09.90 GB 9021175**
**21.03.91 GB 9106048**

(43) Date of publication of application:
**22.03.95 Bulletin 95/12**

(60) Publication number of the earlier application in accordance with Art.76 EPC: **0 550 485**

(84) Designated Contracting States:
**AT BE CH DE DK ES FR GB GR IT LI LU NL SE**

(71) Applicant: **SMITHKLINE BEECHAM BIOLOGICALS s.a.**
**89 rue de l'Institut**
**B-1330 Rixensart (BE)**

(72) Inventor: **Van Wijendale, Frans**
**Teckerstraat 12**
**B-3040 Ottenburg (BE)**
Inventor: **Slaoui, Moncef, SmithKline Beecham Biologicals s.a.,**
**89 rue de l'Institut**
**B-1330 Rixensart (BE)**
Inventor: **Bruck, Claudine, SmithKline Beecham Biologicals s.a.,**
**89 rue de l'Institute**
**B-1330 Rixensart (BE)**
Inventor: **Francotte, Myriam, SmithKline Beecham Biologicals s.a.,**
**89 rue de l'Institute**
**B-1330 Rixensart (BE)**
Inventor: **Kummert, Suzy, SmithKline Beecham Biologicals s.a.,**
**89 rue de l'Institute**
**B-1330 Rixensart (BE)**

(74) Representative: **Dalton, Marcus Jonathan William et al**
**SmithKline Beecham plc**
**Corporate Intellectual Property**
**SB House**
**Great West Road**
**Brentford, Middlesex TW8 9BD (GB)**

(54) **Derivatives of gpl60 and vaccines based on gpl60 or a derivative thereof, containing an adjuvant.**

(57) This invention provides novel, substantially uncleavable forms of gp 160, and vaccine formulations containing gp 160 or a derivative thereof, adjuvanted with 3-D Mpl. The compositions are useful for the immunotherapeutic and immunoprophylatic treatment of HIV infections.

EP 0 644 201 A1

This invention relates to novel vaccine and pharmaceutical formulations and to their manufacture and use in the treatment of AIDS. In particular the invention relates to the use of gp160 or derivative thereof, including novel forms of gp 160 in a vaccine, adjuvanted with 3-D Monophosphoryl lipid A.

Retroviruses, that is, viruses within the family, Retroviridae, are a large family of enveloped, icosohedral viruses of about 150 nm having a coiled nucleocapsid within the core structure and having RNA as the genetic material. The family comprises the oncoviruses such as the sarcoma and leukemia viruses, certain immunodeficiency viruses and the lentiviruses.

Human immunodeficiency virus type 1 (HIV-1) is the etiological agent of acquired immune deficiency syndrome, also known as AIDS. This retrovirus has a complex genetic organisation, including the long terminal repeats (LTRs), the gag, pol, and env genes, and other genes. This retrovirus carries a number of viral antigens which are potential candidates either alone or in concert as vaccinal agents capable of inducing a protective immune response.

The HIV-1 envelope protein is synthesized as a polyprotein precursor which is subsequently glycosylated within infected cells to give a glycoprotein with a mol. weight of 160 kDa (gp160), which is processed by proteolysis into a gp120 external glycoprotein and gp41 transmembrane protein.

A DNA coding region for proviral HIV can be prepared from any of the several immunodeficiency virus genomic clones reported in the literature. See, for example, Shaw et al., Science 226:1165(1984); Kramer et al., Science 231:1580(1986). Alternatively, an immunodeficiency virus genomic clone can be prepared from virus isolated from clinical specimens by standard DNA cloning techniques. See, for example, Gallo et al., U.S. Patent 4,520,113; Montagnier et al., U.S. Patent 4,708,818.

The proviral DNA sequence of HIV-BH1O-2 is described by Ratner et al., "Human Retroviruses and AIDS" 1989, HIV Sequence Database ed. Gerald Meyers, Los Alamos National Laboratory. The sequence is 8932 bp long, the env gene being located at 5580-8150 and the cleavage sites therein at 7088 and 7112 corresponding to amino acids 503 and 511 (cleavage after these residues) (numbering according to Los Alamos database).

According to the present invention, there is provided HIV gp160 which has been modified to provide amino acids other than lysine or arginine at positions 502 and 510 independently.

Suitable replacements for lysine are those similar to lysine in hydrophilicity and size, such as histidine, threonine, serine, asparagine, aspartic acid, glutamine and glutamic acid. Of these, the preferred amino acid is glutamic acid. The preferred protein is both uncleavable due to the mutations introduced and also able of eliciting cross neutralising antibody, which is dependent on the correct folding of the protein.

More preferably the invention provides the modified protein of the invention, in oligomeric form. In particular with a relative molecular weight 640 kDa. Based on the molecular weight of gp160 monomer, this form is believed to be tetrameric. This is advantageous since viral surface proteins naturally exist as oligomers which in-vivo form spikes which protude from the viral surface. As many neutralising epitopes are conformational, it is clearly important to mimic as closely as possible the form of the antigen as it appears naturally, since this will provide the most relevant immune response.

In a preferred enbodiment the invention provides the modified protein of the invention, in an oligomeric and substantially pure form.

By substantially pure form is meant at least 75% pure, more preferably 90% pure, more preferably 99% pure.

In a further aspect the invention provides a process for preparing modified HIV gp160 according to the invention which process comprises expressing DNA encoding said modified protein in a recombinant eukaryotic host cell and recovering the modified protein product.

The DNA polymer comprising a nucleotide sequence that encodes the modified protein also forms part of the invention.

The recombinant DNA molecule of the invention may be prepared in accordance with the invention by the condensation of appropriate mono-, di- or oligomeric nucleotide units.

The preparation may be carried out chemically, enzymatically, or by a combination of the two methods, in vitro or in vivo as appropriate. Thus, the DNA molecule may be prepared by the enzymatic ligation of appropriate DNA fragments, by conventional methods such as those described by D. M. Roberts et al in Biochemistry 1985, 24, 5090-5098.

The DNA fragments may be obtained by digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70°C, generally in a volume of 50μl or less with 0.1-10μg DNA.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 50µl or less. Fragments can be polymerised and amplified by polymerase chain reaction using Taq polymerase (ref. PCR Protocols 1989 - a guide to Methods and Applications, Ed. M.A. Innis et al., Acadamic Press).

Enzymatic ligation of DNA fragments may be carried out using a DNA ligase such as T4 DNA ligase in an appropriate buffer at a temperature of 4°C to ambient, generally in a volume of 50µl or less.

The chemical synthesis of the DNA molecule or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982),or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801. Preferably an automated DNA synthesizer is employed.

DNA polymer which encodes the modified protein may be prepared by site directed mutagenesis of the cDNA which codes for unmodified protein, by conventional methods such as those described by G. Winter et al in Nature 1982, 299, 756-758 or by Zoller and Smith 1982; Nucl. Acids Res., 10, 6487-6500.

The invention also extends to a vector comprising the recombinant DNA molecule of the invention and to a recombinant vaccina virus containing said vector.

The vector may be prepared in accordance with the invention by cleaving a vector to provide a linear DNA segment having a intact replicon, and ligating said linear segment and one or more DNA molecules which, together with said linear segment complete the recombinant DNA molecule of the invention.

The recombinant host cell of the invention may be prepared by transforming a vaccina virus with a vector of the invention.

The modified protein product is isolated from conditioned medium by standard techniques of protein isolation and purification. Detergents e.g., Decyl PEG-300, DO Decyl PEG Triton X100. Thesit Deoxycholate, can be added in order to effect cell lysis and free the modified protein from cell membrane material. Of these Decyl PEG-300 and Thesit Deoxycholate are preferred, DO Decyl PEG Triton X100 Modified protein can then be purified by a series of ultrafiltration steps, ultracentrifugation steps, selective precipitations with e.g., ammonium sulfate or PEG, density gradient centrifugation in CsC1 or sucrose or metrizamide gradients and/or chromatographic steps, such as affinity chromatography, immunoaffinity chromatography preferred, HPLC, reversed phase HPLC, cation and anion exchange, size exclusion chromatography and preparative isoelectric focusing. Purification utilising immunoaffinity chromatography is preferred. During or following purification, the modified protein can be treated with, e.g., formaldehyde, glutaraldehyde or NAE to enhance stability or immunogenicity.

For preparation of the oligomeric form of the invention it is preferred to use mild conditions during the purification steps. In particular, reducing agent should be avoided, and non ionic detergents such as Decyl PEG-300 (polyethyleneglycol 300 monodecylether) are preferred to ionic detergents for cell lysis and solubilisation during the chromatographic steps. A preferred affinity chromatography medium is Lentil lectin Sepharose and a preferred immunoaffinity chromatography medium is an anti-gp160 monoclonal antibody such as 178.1 (WO 90/06358) coupled on a suitable carrier such as glutaraldehyde-activated Trisacryl (IBF).

The modified protein may be adsorbed from the monoclonal antibody in the presence of the detergent octyl glucopyranoside which can be removed by dialysis. The detergents are preferably used at concentrations above their theoretical critical micelle concentration.

The modified protein produced in accordance with this invention is useful as a diagnostic agent for detection of exposure to HIV. The modified protein is also useful in vaccines for the prevention of infection or for the inhibition or prevention of disease progression.

This invention also relates to a vaccine and pharmaceutical compositions containing the modified protein of this invention. Such compositions will contain an immunoprotective quantity of the modified protein of this invention and maybe prepared by conventional techniques.

In the vaccine of the invention, an aqueous solution of the protein can be used directly. Alternatively, the protein, with or without prior lyophilization, can be mixed or absorbed with any of the various known adjuvants. Such adjuvants include, but are not limited to, aluminium hydroxide, muramyl dipeptide and saponins such as Quil A, 3D-MPL (3Deacylated monophosphoryl lipid A), or TDM. As a further exemplary alternative, the protein can be encapsulated within microparticles such as liposomes. In yet another exemplary alternative, the protein can be conjugated to an immuostimulating macromolecule, such as killed Bordetella or a tetanus toxoid.

Vaccine preparation is generally described in New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of the modified protein of the present invention in each vaccine dose is selected as an amount which induces an immunoprotective response without significant, adverse side effects in typical vaccines. Such amount will vary depending upon which specific immunogen is employed and whether or not the vaccine is adjuvanted. Generally, it is expected that each dose will comprise 1-1000 $\mu$g of modified protein, preferably 10-200 $\mu$g. An optimal amount for a particular vaccine can be ascertained by standard studies involving observation of antibody titres and other responses in subjects. Following an initial vaccination, subjects will preferably receive a boost in about 4 weeks, followed by repeated boosts every six months for as long as a risk of infection exists.

The invention further provides modified protein of the invention for use in vaccinating a host and use of modified protein of the invention in the preparation of a vaccine.

In addition to vaccination of persons susceptible to HIV infections, the pharmaceutical compositions of the present invention may be used to treat, immunotherapeutically, patients suffering from HIV infections.

Accordingly, in one aspect of the present invention there is provided a method of treating a human susceptible to or suffering from an HIV infection by administering an effective amount of the modified gp160 as herein described.

Coincidental with the concept of utilising sub unit components produced, for example, by means of recombinant DNA technology, comes the need for adjuvants and/or carriers to present immunogens effectively to the host immune system such that both arms of the immune response (neutralising antibody and effector cell mediated immunity (DTH)) are produced. In the context of the present invention, (ie in the prophylactic or therapeutic treatment of HIV infections) we have discovered than an immunostimulating moiety, 3D MPL is able to stimulate both arms of the immunesystem.

Accordingly in a preferred aspect of the present invention there is provided a pharmaceutical formulation comprising gp 160 or an immunological derivative thereof and 3D Monophosphoryl lipid A (3D-MPL) with a suitable carrier.

In a preferred embodiment of the present invention the gp 160 or an immunological derivative thereof and 3D- MPL are presented in an oil in water emulsion. This system provides enhanced neutralising activity.

Accordingly in a preferred aspect of the present invention there is provided a pharmaceutical or vaccine formulation comprising gp 160 or an immunological derivative thereof,3D-MPL in an oil in water carrier, said carrier comprising an emulsion of a tetrapolyol and a non toxic mineral oil in a buffered saline solution.

Preferably the carrier comprises a Pluronic polyol such as Pluronic L121, and squalane or squalene or other metabolisable oils An emulsifer such as Tween 80 or Tween 28 is preferably provided to stabilise the emulsion.

The carrier preferably contains only submicron particles of between 100 and 400 nm.

The concentration of antigen in the final formulation is preferably between 10$\mu$g to 150$\mu$g/ml, more preferably between 20 $\mu$g to 100$\mu$g/ml.

The concentration range of adjuvant, 3D-MPL, in the vaccine, is preferably between 10$\mu$g to 100$\mu$g/ml more preferably between 25 to 50$\mu$g/ml.

The present invention further provides the vaccine formulations as herein described for use in medicine, in particular for use in the treatment by immonotherapy and prophylatic treatment of HIV-1 infections such as AIDS or AIDS related complex (ARC).

In a further aspect of the present invention there is provided a method of producing a vaccine comprising gp160 or an immunological derivative thereof, 3D monophosphoryl lipid A with a suitable carrier, the method comprising mixing gp160 or immunological derivative with said carrier and with 3D monophosphoryl lipid A.

In an embodiment, there is provided a method of producing a vaccine as herein described in an oil in water carrier wherein an oil in water emulsion is microfluidized to provide sub micron particles in said emulsion and mixed with gp160 or immunological derivative thereof and 3D MPL.

Typically the 3D-MPL is premixed with the emulsion, thereafter the antigen is mixed into the resulting composition.

3D-MPL may be obtained by the methods disclosed in British Patent 2211502.

Suitable carriers in this context, comprise oil in water emulsions. The formulations of the present invention provide enhanced neutralising titres when compared with conventional vaccine formulations comprising, alum alone as the adjuvant (the only adjuvant licensed for human use).

The term immunological derivatives is used herein to include immunogenic fragments of gp 160 which when adjuvanted with 3D Monophosphoryl lipid A are capable of raising neutralising antibodies against HIV-1. As such this will include, for example the HIV-1 outer membrane glycoprotein gp 120, modified gp 160 as herein described as well as the naturally occurring isolated gp 160. Particularly preferred are those derivatives which are also able to raise a DTH response.

Thus most preferably the invention provides the modified form of the protein in oligomeric form which when purified under gentle, non-reducing conditions is shown to have an apparent molecular weight of between 600-700 KDa 640 Kd and is believed to be a tetramer. This tetramer may be destabilised by running the protein on SDS gel under non-reducing conditions, which then provides a dimer of 330 kd and a monomer. The dimer may further be reduced under standard reducing conditions to yield the monomer.

All these forms of the gp160 may be used in the formulations of the present invention.

The production of gp 160 or derivatives thereof may be achieved by methods known in the art. Typically this will involve the cloning and expression of the gene encoding for gp 160 in a suitable host. The production of recombinant gp 160 (rgp 160) in such ways may be achieved using the techniques described in Maniatis et al; Molecular Cloning - A laboratory Manual; Cold Spring Harbour 1982.

A variety of eukaryotic cells and expression systems are available for expression of the recombinant DNA molecules. The most widely used among these are yeast, insect and mammalian systems, although the invention is not limited to use of these. Such systems employ a recombinant DNA molecule of the invention, optionally a selection marker and, in some cases, maintenance functions such as an origin of replication.

Insect cells which can be used in the invention include Drosophila cells and Lepidoptera cells. Useful Drosophila cells include S1, S2, S3, KC-O and D. hydei cells. See, for example, Schneider et al., J. Embryol. Exp. Morph. 27:353 (1972); Schulz et al., Proc. Natl. Acad. Sci USA 83:9428 (1986); Sinclair et al., Mol. Cell. Biol. 5:3208 (1985).

Drosophila cells are transfected by standard techniques, including calcium phosphate precipitation, cell fusion, electroporation and viral transfection. Cells are cultured in accordance with standard cell culture procedures in a variety of nutrient media, including, e.g., M3 media which consists of balanced salts and essential amino acids. See, Lindquist, DIS 58:163 (1982).

Promoters known to he useful in Drosophila include mammalian cell promoters such as SV40 as well as Drosophila promoters, the latter being preferred. examples of useful Drosophila promoters include the Drosophila metallothionein promoter, the 70 kilodalton heatshock protein promoter (HSP70) and the COPIA LTR. See, for example, DiNocera et al., Proc. Natl. Acad. Sci. USA 80;7095 (1983); McGarry et al., Cell 42:903 (1985).

Useful Lepidoptera cells include cells from Trichoplusia ni, Spodoptera frugiperda, Heliothis zea, Autographica californica, Rachiplusis ou, Galleria melonella, Manduca sexta or other cells which can be infected with Baculoviruses, including nuclear polyhedrosis viruses (NPV), single nucleocapsid viruses (SNPV) and multiple nucleocapsid viruses (MNPV). The preferred Baculoviruses are NPV or MNPV Baculoviruses because these contain the polyhedrin gene promoter which is highly expressed in infected cells. Particularly exemplified hereinbelow is the MNPV virus from Autographica californica (AcMNPV). However, other MNPV and NPV viruses can also be employed the silkworm virus, Bombyx mori. Lepidoptera cells are co-transfected with DNA comprising the recombinant DNA molecule of the invention and with the DNA of an infectious Baculovirus by standard transfection techniques, as discussed above. Cells are cultured in accordance with standard cells culture techniques in a variety of nutrient media, including, for example, TC100 (Gibco Europe; Gardiner et al., J. Inverteb. Pathol. 25:363 (1975) supplemented with 10% fetal Calf serum (FCS). See, Miller et al., in Setlow et al., eds., Genetic Engineering: Principles and Methods., Volume 8, New York, Plenum, 1986, pages 277-298.

Promoters for use in Lepidoptera cells include promoters from a Baculovirus genome. The promoter of the polyhedrin gene is preferred because the polyhedrin protein is naturally over expressed relative to other Baculovirus proteins. The polyhedrin gene promoter from the AcMNPV virus is preferred. See, Summers et al., TAES Bull. NR 1555, May 1987; Smith et al., EP-A-127,839; Smith et al. Proc. Natl. Acad. Sci. USA 82:8404(1985); and Cochran, EP-A-228,036.

For expression in mammalian cells, the recombinant DNA molecule is likewise cloned within a cloning vector and is then used to transfect the mammalian cells. The vector preferably comprises additional DNA functions for gene amplification, e.g., a DHFR expression cassette, and may also comprise additional functions for selection and/or amplification, e.g., a neomycin resistance cassette for G418 selection. Other functions, such as for transcription enhancement can also be employed. Yet other functions can be comprised within the vector for stable episomal maintenance, if desired, such as maintenance functions of Bovine Papilloma Virus. Alternatively and preferably the cloning vector is a recombinant mammalian virus such as vaccinia virus.

Vaccinia virus is a particularly useful vector in that recombinants can be readily constructed by integration of the foreign gene in a nonessential region of the vaccinia DNA and thus retain infectivity. When properly engineered the proteins are synthesized, processed, and transported to the membrane of infected cells. Although vaccinia virus infection leads to cell death, there is little lysis and the majority of cells remain intact, allowing easy extraction of the required protein from infected cells. A vaccinia expression system has been developed by Barrett et al., Aids Research and Human Retroviruses 1989; 5: 159-171.

Useful mammalian cells include cells from Chinese hamster ovary (CHO), NIH3T3, COS-7, CV-I, BHK-21, mouse or rat myeloma, HAK, Vero, HeLa, human diploid cells such as MRC-5 and WI38, or chicken lymphoma cell lines, CV-I and BHK-21 being preferred.

Transfection and cell culture are carried out by standard techniques. Production in mammalian cells can also be accomplished by expression in transgenic animals.

Regulatory sequences useful to drive gene expression in mammalian cell lines or mammalian primary cells include the SV 40 early and late gene promoters, the metallothionein promoter, viral LTR's such as the Rous sarcoma LTR, the Moloney sarcoma virus (MSV) LTR or the mouse mammary tumor virus (MMTV) LTR, or the adenovirus major late promoter and hybrid promoters such as a hybrid BK virus and adenovirus major late promoter. The control elements region can also comprise downstream functions, such as regions for polyadenylation, or other functions, such as transcription enhancer sequences.

Yeasts which can be used in the practice of the invention include those of the genera Hanensula, Pichia, Kluveromyces, Schizosaccharomyces, Candida and Sacchoromyces. Sacchoromyces cerevisiae is the preferred yeast host. Useful promoters include the copper inducible (CUP1) promoter, glycolytic gene promoters, e.g., TDH3, PGK and ADH, and the PHO5 and ARG3 promoters. See, e g. Miyanohara et al., Proc. Nat1. Acad. Sci. USA 80:1 (1983); Mellor et al., Gene 24:1 (1983); Hitzeman et al., Science 219:620 (1983); Cabezon et al., Proc. Nat1. Acad. Sci. USA 81:6594 (1984).

Example 1

(i) Expression of modified gp160 in CV-I cells

The HIV env gene of the BH10 molecular clone was mutagenized to abolish precursor cleavage.
For this, the "cleavage sequences" lys ala lys arg and arg glu lys arg present in these sequences were modified by site directed mutagenesis to lys/arg x glu arg.

```
                        cleavage site 1              cleavage site 2
                        (position 502)               (position 510)
                             ↓                               ↓
BH10          AAG GCA AAG AGA AGA GTG GTG CAG AGA GAA AAA AGA
Clone:        LYS ALA LYS ARG ARG VAL VAL GLN ARG GIU LYS ARG


was mutated                  ↓                               ↓
to:           AAG GCA GAG AGA AGA GTC GTG CAG AGA GAA GAA AGA
              LYS ALA GLU ARG ARG VAL VAL GLA ARG GLU GLU ARG
```

The complete mutagenized env gene (nucleotides 5802-8478) was cloned into the vaccinia plasmid transfer vector pGS20 [Mackett M.G., Smith L. and Moss B. (1984) J. Virology 49: 857-864] and transferred into infectious vaccinia virus by recombination. Recombinant vaccinia plaques were screened for env expression by capture EIA and positive plaques recloned twice on CV-1 cells. The production of uncleaved gp160 in these cell lines was further verified by RIPA after metabolic labelling of cells infected with recombinant vaccinia. Cell surface expression was confirmed by fluorescent labelling of the intact cell surface using anti-gp120 antibodies.

(ii) Purification of Modified gp160 from Anchorage dependent cells.

The HIV gp160 envelope protein from (i) was purified in a three step protocol: lysis of the host cells and extraction of the antigen with the aid of a detergent followed by two affinity chromatography steps. All the purification steps were performed at 4 °C.

Step 1: lysis of the CV-1 cells and extraction of the antigen gp160

After thawing, the cell suspension and microcarrier beads corresponding to 20 l of culture were centrifuged at 2,200 xg for 15 min and the supernatant was discarded. The precipitate was resuspended in 1 l of 30 mM tris/HCl buffer pH 8 supplemented with 150 mM NaCl, 1% polyethyleneglycol 300 monodecylether (Decyl PEG) and 20 mcg/ml aprotinin. The cells were lysed for 1 hour on ice with occasional shaking by hand and centrifuged at 11,300 xg for 20 min. After separation of the supernatant, the pellet was washed with 400 ml of lysis buffer and centrifuged at 11,300 xg for 20 min. The cell debris and microcarrier beads were discarded and the combined supernatants were used for further purification.

Step 2: Affinity chromatography on Lentil lectin Sepharose 4B

The lysate was chromatographed on a Lentil lectin Sepharose 4B (Pharmacia-LKB) column (2.5 cm x 20 cm) equilibrated with 30 mM Tris/HCl buffer pH 8 supplemented with 150 mM NaCl and 0.1% Decyl PEG. After loading the lysate at a flow rate of 1 ml/min, the column was washed with 30 mM Tris/HCl buffer pH 8 supplemented with 1 M NaCl and 0.1% Decyl PEG. Subsequently, the antigen was eluted from the column with 0.5 M methyl $\alpha$-D-mannopyranoside in equilibration buffer and gp160 positive fractions were pooled. The wash and elution steps were carried out at a flow rate of 5.5 ml/min.

Step 3: Immunoaffinity chromatography on 178.1-Trisacryl

The anti-gp160 monoclonal antibody 178.1 (Patent publication No. WO 90/06358) was purified from ascites fluid on a Protein G-Sepharose column (Pharmacia-LKB) and subsequently coupled on glutaraldehyde-activated Trisacryl (IBF) according to the manufacturer's guidelines. The antibody, which is directed against an epitope on the gp120 moiety of the antigen ($V_3$ loop), has been coupled at a density of 1.5 mg/ml gel.

The resin was packed into a column (2.5 cm x 10 cm) and equilibrated in 30 mM Tris/HC1 buffer pH 8 supplemented with 150 mM NaCl and 0.1% Decyl PEG.

The Lentil lectin Sepharose 4B eluate was loaded onto the column by overnight recycling at 1 ml/min. Subsequently the column was washed at a flow rate of 3.3 ml/min with 20 column volumes of 30 mM Tris/HC1 buffer pH 8 supplemented with 1 M NaCl and 1% n.octyl $\beta$-D-glucopyranoside (OGP). Finally, the antigen was eluted at the same flow rate with 0.1 M citric acid buffer pH 3.3 supplemented with 1% OGP. Elution fractions were immediately neutralized with 1 M Tris/HC1 pH 8.8 and the antigen positive fractions were pooled.

(iii) Protein determination

Protein concentrations were determined by the method of Bradford (Bradford, 1976) using bovine serum albumin as the standard.

(iv) Antigen determination

The amount of gp160 antigen was measured by an in-house developed sandwich ELISA using sheep anti-gp41 as capturing monoclonal antibody and murine anti-gp120 as indicator monoclonal antibody. Further detection was with a classical biotinylated anti-mouse antibody, streptavidin and peroxidase system.

(v) Polyacrylamide gel electrophoresis and Western blotting

SDS-slab gel electrophoresis was carried out in 10% polyacrylamide gels according to the method of Laemmli (Laemmli, 1970). After migration, proteins were visualized by silver staining after periodic acid oxidation (Pas staining) (Dubray et al, 1982).

Electrophoretic runs were carried out in the presence and in the absence of a reducing agent. Protein bands were further identified by Western blotting on nitrocellulose according to Towbin (Towbin et al, 1979) and probing was with antibodies either directed against the gp160 antigen or against host cell (CV-1) or vaccinia proteins.

(vi) Leakage of 178.1 monoclonal antibody

The presence of monoclonal antibody 178.1 in the purified gp160 antigen was measured by ELISA. Antibodies were captured by a goat-anti-mouse antibody and detected with a biotinylated anti-mouse antibody. Further detection was with the streptavidin-peroxidase system.

(vii) Size exclusion chromatography of pure env gp160

Purified gp160 was chromatographed on a TSK 4000 SW HPLC column (7.5 mm x 300 mm) equilibrated in 0.2 M phosphate buffer pH 7 supplemented with 1% OGP. Flow rate was 0.75 ml/min and column fractions were analysed for antigen by ELISA.

(viii) Purity determination

Purity was estimated after each purification step by SDS-polyacrylamide gel electrophoresis under reducing conditions. Protein bands were visualized by PAS staining and further identified by Western blotting.

The results clearly showed the antigen to be free of detectable contaminating proteins after chromatogrphy on the immunoaffinity column. However the final product might be contaminated with trace amounts of monoclonal antibody leaking from the immunoaffinity column. Therefore, the amount of 178.1 antibody in the pure gp160 was measured by ELISA and ranged from 0.004% to 0.02% of the total amount of protein present in the samples.

The purified antigen was analysed for the presence of oligomeric forms of gp160. The formation of oligomers could reflect the structure of gp160 in the virus where the envelope proteins are arranged as spikes at the surface of the virion. The presence of cysteines in the primary sequence of the antigen allows formation of (homo-)oligomers linked by disulfide bridges. This was demonstrated by SDS-polyacrylamide gel electrophoresis in the presence and in the absence of $\beta$-mercaptoethanol. Without a reducing agent, the antigen showed protein bands of molecular mass larger than 160,000 Da, even in the presence of detergent (SDS). In contrast, when the antigen was boiled in the presence of a reducing agent, a single protein band at 160 kDa was observed. In order to determine the size of the oligomeric structures, the antigen was analysed by HPLC size exclusion chromatography. It followed from column calibration with standards of known molecular mass that most of the gp160 molecules eluted at a retention time corresponding to a molecular mass of 640,000 Da. Therefore, it was concluded that in the presence of a detergent but without a reducing agent, most of the HIV gp160 env protein assembled into tetrameric structures.

(ix) Analysis

The final product shows an antigen/protein ratio of about 2 which fits the ELISA content of the lysate (0.8-1.4 mg antigen by ELISA per liter culture).

Example 2

Expression of modified gp160 in BHK-21 and purification of env. gp160 from cells cultured in suspension

The HIV gp160 envelope protein expressed in BHK-21 cells by an analogous method to that described in Example 1(i) with a recombinant vaccinia virus was purified according to the scheme outlined in Example 1 (ii) except for some minor modifications at the lysis step.

Step 1: lysis of the BHK-21 cells and extraction of the antigen gp160

After thawing, the cell suspension ($10^9$ cells) was centrifuged for 15 min at 2,200 xg band the supernatant was discarded. The cell pellet was resuspended in 50 ml of 30 mM Tris/HCl buffer pH 8 supplemented with 150 mM NaCl, 1% polyethyleneglycol 300 monodecylether (Decyl PEG) and 20 mcg/ml aprotinin. The cells were lysed for 1 hour on ice with occasional shaking by hand and centrifuged at 11,300 xg for 15 min. After separation of the supernatant, the pellet was washed with 20 ml of lysis buffer and centrifuged at 11,300 xg for 15 min. The pellet was discarded and the combined supernatants were used for further purification.

Step 2 and 3 and subsequent analysis was carried out as described in Example 1 (ii)-(ix).

Example 3

Preparation of gp160 - 3D-MPL Vaccines formulations

3 (a) rgp160-Aluminium Hydroxide plus 3D-MPL

Purified rgp160 (100 $\mu$g or 20 $\mu$g each per dose) from vaccina (example 1) was adsorbed overnight at 4°C on aluminium hydroxide (alum) corresponding to 0.5 $\mu$g equivalents $Al^{3+}$ in 1 ml of 150 mM NaCl, 10 mM phosphate buffer pH 6.8. After overnight incubation, the adjuvant preparation was centrifuged and the supernatant removed. An equal volume of adsorption buffer containing 100 $\mu$g 3D-MPL was then added to the alum-bound rgp160. More than 95% of the rgp160 was found to be adsorbed on aluminium hydroxide.

3 (b) rgp160-3D-MPL oil in water emulsion

The vehicle was prepared as follows. Pluronic L121 5% (BASF Wyandotte, New Jersey) (v/v) and 10% squalane (Aldrich) were added to phosphate-buffered saline (PBS) containing 0.4% (v/v) Tween 80. This mixture was then microfluidized. For microfluidization, the emulsion was cycled ten times through a microfluidizer (Model M110 Microfluidics Corp., Newton, Mass.). The resulting emulsion comprised only submicron particles. One volume of this emulsion was mixed to an equal volume of twice concentrated rgp160 (either 20 $\mu$g or 100 $\mu$g) and vortexed briefly to ensure complete mixing of the components. 100 $\mu$g/ml 3D-MPL was then added to this rgp160 o/w emulsion. The final preparation consisted of 0.2% Tween 80, 2.5% Pluronic L121, 5% squalane, 100 $\mu$g 3D-MPL and rgp160 (100 $\mu$g or 20 $\mu$g) in a 1 ml injection dose.

Example 4

Immunogenicity in guinea pigs

ELISA and neutralization titers

Five guinea pigs were immunized with 3 injections of 50 $\mu$g modified gp160 (Example 1) in SAF-1 (Syntex adjuvant formulation-1) Byars NE and Allison A.C. (1987) Vaccine 5: 223-227 at monthly interval. The sera were tested 2 weeks and 1 month after secondary immunization, as well as 2 weeks after tertiary immunization of the guinea pigs. The results are described in Table 2.

A capture enzyme immunoassay (EIA) based on a lysate of HIV-1 (IIIB) infected cells was used to determine the ELISA titer of the antisera after the first and second boost. The test used is very similar to that published by Moore et al. [Moore J.P. et al., 1989, AIDS 3:155 (63)].

The microplate neutralization assay is based on the detection of HIV gag antigen in indicator cells. Briefly, SupT1 cells (Hecht et al., 1984, Science 226:1445) are used as indicator cells. The viral inoculum consists of cell free supernatant of a HIV-1 (IIIB) producing lymphoid cell line. The supernatant is centrifuged at high speed to eliminate cells and cell debris, aliquoted in 1 ml vials and stored at -80°C until use. The sera to be tested are inactivated at 56°C for 30 min. prior to testing. Our negative control consists of a pool of sera from preimmune or adjuvant alone inoculated animals (same species as the sera to be tested). For neutralization, 750 $TCID_{50}$ are incubated with serial two fold dilutions of the sera for 1 hour at 37°C. SupT1 cells are then added ($4.10^4$ cells/well) and incubated 4 days at 37°C. The cytopathic effect is microscopically monitored, Triton X-100 (1 % final concentration) is added to each well and the plate is frozen. A sandwich ELISA is used to monitor the relative amount of viral antigen produced in the cultures. The plates are coated with an anti p55 monoclonal antibody. The above Triton X-100 treated samples are incubated in the plate and the presence of gag antigen is visualized by biotinylated HIV-1 + human IgGs followed by a streptavidin peroxidase step. The percentages of reduction of HIV-1 antigen production relative to the control are then evaluated for all the serum dilutions tested. Using a curve fit to the data points by non linear least squares analysis, the serum dilution (if any) giving a 50% reduction in antigen production compared to control, is extrapolated.

Table 2 shows the neutralizing antibody titer after tertiary immunization.

Neutralization titers observed after the second boost are exceeding those found in sera from infected humans. More precisely, the neutralizing titer of our antisera towards the HIV IIIB isolate is on average 4-fold higher than that observed for a group of 5 seropositive WHO reference sera (McKeating et al., 1989, J. Gen. Virol. 70:3326-3333). Neutralization of a series of HIV-1 isolates (cross neutralization) was tested by Dr. Weiss' laboratory (Chester Beatty Laboratories, U.K.) using a more stringent neutralization test that yields a lower sensitivity and titre. The results, described in Table 3 were reproduced on 3 occasions with 2 different bleeds and show good cross neutralization of a variety of HIV-1 strains including an African strain (CBL-4) (see table 3).

Sera from guinea pigs immunized with vaccinia gp160 (Example 1) (01-05) show good cross neutralizing titers after tertiary immunization.

Because of the strong cross neutralizing response observed after 3 doses, vaccinia gp160 of the invention is considered of potential use for HIV-1 vaccination.

Example 5

Study of the effect of different vaccine formulations on the immunogenicity of purified HIV vaccinia recombinant gp160 (IIIB isolate) in Rhesus monkeys

In this study, the ability of different vaccine formulations to enhance the immunogenicity of purified vaccinia recombinant gp160 (rgp160) was evaluated in Rhesus monkeys (Macaca mulatta). Adjuvants tested were aluminium hydroxide (Alhydrogel, Superfos - Denmark) in combination with 3D-MPL (Example 2b) (3D Monophosphoryl Lipid A, Ribi); 3D-MPL in oil in water emulsion (Example 2a).

Rhesus monkeys (Macaca mulatta) weighing 3.5 to 5 kg were randomly assigned into seven groups containing 3 or 4 animals per group.

Groups were immunized with different doses of rgp160 formulated in 3 adjuvant formulations, as follows:

Group 1 (4 monkeys) :100 $\mu$g rgp160 adsorbed on aluminium hydroxide plus 3D-MPL
Group 2 (4 monkeys) :20 $\mu$g rgp160 adsorbed on aluminium hydroxide plus 3D-MPL
Group 3 (4 monkeys) :100 $\mu$g rgp160 plus 3D-MPL in o/w emulsion
Group 4 (4 monkeys) :20 $\mu$g rgp160 plus 3D-MPL in o/w emulsion

5.1. Antigen-Adjuvant preparations

All the rgp160 formulations were prepared immediately before use. Each injection dose was administered in a 1 ml volume.

Groups of rhesus monkeys were injected intramuscularly in the brachial triceps with 1 ml dose of various gp160 formulations at day 0 and day 35. Two weeks after the second immunization, the animals were bled for antibody determinations.

5.2. Read-out

The sera taken from these animals 2 weeks after the second injection were tested in ELISA and neutralization assays

5.3. ELISA

A modification of the capture immunoassay whose general protocol described in Thiriart et al (Thiriart et al, 1989, J. Immunol 148 6:832-1836) was used This assay uses a commercial monospecific anti gp41 reagent from Biochrom. A crude lysate of vaccinia gp160 infected BHK 21 cells is used as antigen.

5.4. Neutralization assay

The microplate neutralization assay is based on the detection of HIV gag antigen in indicator cells. Briefly, SupT1 cells (Hecht et al, 1984, Science 226:1445) are used as indicator cells. The viral inoculum consists of cell free supernatant of HIV-1 (IIIB) producing lymphoid cell line. The supernatant is centrifuged at high speed to eliminate cells and cell debris, aliquoted in 1 ml vials and stored at - 80°C until use. The sera to be tested are inactivated at 56ºC for 30 min. prior to testing. The negative control consists of a pool of sera from preimmune animals. For neutralization, 750 $TCID_{50}$ are incubated with serial two fold dilutions of the sera for 1 hour at 37°C. SupT1 cells are then added ($2.10^4$ cells/well) and incubated 4 days at 37°C. The cytopathic effect is microscopically monitored, Triton X-100 (1% final concentration) is added to each well and the plate is frozen. A sandwich ELISA is used to monitor the relative amount of viral antigen produced in the cultures. The plates are coated with an anti p55 monoclonal antibody. The above Triton X-100 treated samples are incubated in the plate and the presence of gag antigen is visualized by biotinylated HIV-1 + human IgGs followed by a streptavidin peroxydase step. The percentages of reduction of HIV-1 antigen production relative to the controls are then evaluated for all the serum dilutions tested. Using a curve fit to the data points by linear regression analysis, the serum dilution (if any) giving a 50% reduction in antigen production compared to controls, is extrapolated.

5.5. Results

Table 4 shows the ELISA and neutralizing antibody titer after the second immunization. The general immunogenicity of HIV rgp160 in 3D-MPL o/w is very good. When doses of 20 $\mu$g of gp160 are administered to the animals, the neutralising titre (NT) and ELISA titers observed were extremely good in the group of animals receiving rgp160 in 3D-MPL o/w (group 4). These data suggest a superior adjuvant effect of the 3D-MPL o/w emulsion. The immune response of animals immunized with gp160 adsorbed on Alum in the presence of 3D-MPL is poor although some, neutralising antibody is produced. The HIV gp160 contains an hydrophobic moiety and this probably confers the molecule a high affinity for lipids. The HIV rgp160 is best presented to the immune system by using an oil in water based formulation containing 3D-MPL.

Example 6

6.1 Experimental design

Two chimpanzees (No.1, No.2) were immunized with purified vaccinia recombinant gp160 (100 $\mu$g/dose)(rgp160) from example 1.

Two chimpanzees (No.3, No.4) were immunized with purified recombinant gp120 (100 $\mu$g/dose) expressed in Drosophila Schneider cells (rgp120) (Culp et al., Biotechnology, 9:173-177, 1991).

The recombinant proteins were formulated with 3D MPL (100 $\mu$g/dose) in an o/w emulsion. The formulation consisted of 0.2% Tween 80, 2.5% Pluronic L121, 5% squalane, 100 $\mu$g 3D MPL and recombinant antigen dose (100 $\mu$g for rgp160 or rgp120; in a 1 ml injection volume.

Animals were immunized intramuscularly in a leg at month 0, 1 and 2.

Animals were bled every two weeks for antibody determination by ELISA and neutralization assays (see Example 4.3 for Elisa methodology); the HIV neutralization assay was slightly modified, as described below). The induction of delayed-type hypersensitivity response (DTH) was also evaluated, as described below.

6.2 Induction of humoral immunity

As shown in Table 5, chimpanzees vaccinated either with rgp160 or rgp 120 delivered in a 3D MPL o/w emulsion produced high ELISA and neutralizing titers after 3 immunizations. Neutralizing activity could be detected in 3 sera out of 4 after 2 injections and in 4 out of 4 after a further boost. An increase of the neutralizing titers was observed following a third immunization. The ELISA and neutralizing titers measured after this boost are very similar to those obtained in rhesus monkeys immunized three times with the same rgp160 formulation.

6.3 Induction of DTH

DTH tests were performed two weeks after the third immunization. All injections were administered in the belly, in a 100 $\mu$l volume per injection.

The two rgp160 immunized chimps and the two rgp120 treated animals wre skin tested with 3 different antigens (rgp160, rgD26, tetanus toxoid) and 2 control buffers (rgD2t control buffer (PBS) and rgp160 control buffer). Different recombinant antigen doses were tested: 40, 20 or 10 $\mu$g for rgp160 and 20, 10 or 5 $\mu$g for rgD2t.

DTH responses were monitored 24 hrs later by measure of skin thickness. Results are illustrated in Figures 1-3.

A specific DTH response against tetanus toxoid and rgp160 was observed in animals vaccinated either with rgp160 (Fig. 1) or with rgp120 (Fig. 2).

These results indicate that formulations containing 3D MPL in an o/w emulsion are able to induce a specific T cell response (see Table 6).

In conclusion, results obtained in chimpanzees clearly indicate that adjuvant formulations containing 3D MPL in an oil in water emulsion significantly improve humoral (neutralizing antibodies) and effector cell mediated (DTH) immune responses in primates.

Neutralization assay

The microplate neutralization assay is based on the visual evaluation of CPE induced by HIV1 infection in indicator cells. Briefly, SupT1 cells (Hecht et al, 1984, Science $\underline{226}$:1445) are used as indicator cells. The viral inoculum consists of cell free supernatant of HIV-1 (IIIB) producing lymphoid cell line. The supernatant is centrifuged at high speed to eliminate cells and cell debris, aliquoted in 1 ml vials and stored at -80°C until use. The sera to be tested are inactivated at 56°C for 30 min. prior to testing. Our negative control consists of a pool of sera from preimmune animals. For neutralization, 750 $TCID_{50}$ are incubated with serial two fold dilutions of the sera for 1 hour at 37°C. SupT1 cells are then added ($2.10^4$ cells/well) and incubated 4 days at 37°C. The cytopathic effect is microscopically monitored on day 4 and the neutralizing titers are visually determined. The given neutralization titers correspond to the reciprocal of the dilution of serum giving 80% reduction of syncytia formation as compared to preimmune controls. The visually determined titers are further objectivated on day 7 by measuring cell viability in each well using the MTT assay described by Pauwels et al (J. Virol. Methods $\underline{20}$:309-321, 1988). The titers determined in this assay represent the reciprocal of the serium dilution giving 80% protection against CPE as compared to uninfected cells. The visually and MTT determined titers are very reproducible from one assay to another and the MTT determined titers (not shown) are 2 to 4 fold lower than the visually determined ones.

Table 1

| Purification of HIV gp160 expressed in CV-1 and in BHK-21 cells | | | | |
|---|---|---|---|---|
| Purification step | Volume (ml) | Total Protein(mg) | Protein recovery(%) | Antigen recovery(%) |
| CV-1 (1) | | | | |
| Lysis | 1400 | 2000 | 100 | 100 |
| Lentil lectin | 350 | 100 | 5 | 80 |
| Immunoaffinity | 60 | 8 | 0.4 | 65 |
| BHK-21 (2) | | | | |
| Lysis | 1480 | 2688 | 100 | 100 |
| Lentil lectin | 495 | 113 | 4 | 79 |
| Immunoaffinity | 60 | 7 | 0.3 | 58 |

(1) : 20 1 culture as starting material
(2) : 14 1 culture (2.1 x $10^{10}$ cells) as starting material

## Table 2

### IMMUNOGENICITY OF gp160 IN GUINEA PIGS

| Antigen | Dose | Adjuvant | Guinea pig n° | ANTI-HIV EIA titer (1) | | | NEUTRALIZING TITER(IIIB) | | |
|---|---|---|---|---|---|---|---|---|---|
| | | | | Post II | | Post III | Post II | | Post III |
| | | | | 15 d | 1 month | 15 d | 15 d | 1 month | |
| gp160 VACCINE (Example 1) | 50 ug | SAF-1 | 01 | 21653 | 20327 | 158589 | 183 | < 100 | 2207 |
| | | | 02 | 22348 | 16694 | 179971 | 165 | 117 | 1930 |
| | | | 03 | 25360 | 20327 | 151254 | 445 | 394 | 6630 |
| | | | 04 | 35905 | 26175 | 161124 | 353 | 195 | 2034 |
| | | | 05 | ND | 8658 | 77884 | 133 | < 100 | 677 |
| | | | GMT | 25764 | 17330 | 140203 | 229 | 117 | 2079 |

(1)    Represents the inverse of the serum dilution which gives an O.D of 0.5.

EP 0 644 201 A1

Table 3

| SAMPLE I.D. | IIIb Homologous challenge | Heterologous challenge | | | |
|---|---|---|---|---|---|
| | | SF2 | MN | RF | CBL-4 |
| 01 02/05/90 | 80 | 10/<10 | - | - | - |
| 02 02/05/90 | <40 | 80 | 10 | - | 10 |
| 03 02/05/90 | 80 | 20 | - | - | 10 |
| 04 02/05/90 | 40 | 10 | - | - | 10 |
| 05 02/05/90 | 80 | 40 | 10 | - | 10 |
| 01 14/05/90 | 40 | 20 | - | - | 10 |
| 02 14/05/90 | 80 | 20 | - | - | 10 |
| 03 14/05/90 | 160 | 20 | 10 | - | 10/<10 |
| 04 14/05/90 | 20 | 10 | - | - | 40 |
| 05 14/05/90 | 40 | 40 | 0/<10 | - | 10 |
| HUMAN POSITIVE[1] | 160 | 320 | 80 | 160 | 80 |
| HUMAN NEGATIVE | - | - | - | - | - |
| ° Neutralising activity of Guinea Pig Sera to gp160 towards homologous (IIIb) and heterologous isolates | | | | | |

1 Positive neutralizing effect is 90% inhibition of syncytia formation as judged visually.

## Table 4:

IMMUNOGENICITY OF RECOMBINANT VACCINIA gp160 IN RHESUS MONKEYS

| GROUP | ADJ/DOSE | IDENTIFICATION | 50% NT* | ELISA** midpoint titer |
|-------|----------|----------------|---------|------------------------|
| | | ELISA AND NEUTRALIZATION TITERS 15 Days Post 11 | | |
| 1 | ALUM 3D MPL/100µg | IP 111 | < 100 | 576 |
| | | IP 123 | < 100 | 301 |
| | | IP 151 | < 100 | 411 |
| | | IP 167 | < 100 | 630 |
| | | GMT | | 460 |
| 2 | ALUM 3D MPL/20µg | IP 101 | < 100 | 1871 |
| | | IP 103 | < 100 | 1641 |
| | | IP 105 | < 100 | 1437 |
| | | IP 196 | < 100 | 94 |
| | | GMT | | 802 |
| 3 | 3D MPLOW 100µg | IP 120 | 486 | 9043 |
| | | IP 125 | 282 | 5537 |
| | | IP 150 | 285 | 3981 |
| | | IP 152 | 918 | 10629 |
| | | GMT | 435 | 6789 |
| 4 | 3D MPLOW 20µg | IP 114 | 462 | 5413 |
| | | IP 117 | 576 | 5220 |
| | | IP 118 | < 100 | 2529 |
| | | IP 197 | 804 | 5268 |
| | | GMT | 322 | 4404 |

\* Correspond to the reciprocal of the serum dilution giving 50% reduction of the antigen production as compared to the controls

\*\* Correspond to the reciprocal of the serum dilution giving an absorbance equal to 50% of the maximal absorbance value (midpoint titer)

GMT = Geometric Mean Titer

EP 0 644 201 A1

Table 5

ANTI-HIV ANTIBODY RESPONSE IN RGP160 AND RGP120 VACCINATED CHIMPANZEES

| | | Post I | Post II | | | Post III | | |
|---|---|---|---|---|---|---|---|---|
| | | 28 days | 14 days | | 28 days | 14 days | | 28 days |
| Vaccine | Chimp | Neut.Titer | Elisa Titer | Neut.Titer | Neut.Titer | Elisa Titer | Neut.Titer | Neut.Titer |
| rgp160(100µg) | 1 | <50 | 2592 | <50/ | 50/ | 16509 | 200 | 400 |
| 3D MPL o/w | 2 | <50 | 9884 | 100 | 100 | 15665 | 200/ | 400 |
| rgp120(100µg) | 3 | <50 | 3059 | 100 | 100 | 22679 | 800 | 800 |
| 3D MPL o/w | 4 | <50 | 506 | <50/ | 50 | 3917 | 200 | 200/ |

ELISA TITER:  corresponds to the reciprocal of the serum dilution giving an absorbance equal
to 50% of the maximal absorbance value (midpointer titer).

NEUT. TITER:  corresponds to the reciprocal of the serum dilution giving 80% protection from
cytopathic effect of IIIB HIV1 isolate.

/:  slash indicates that the neutralizing titer lies between 2 successive dilutions
of the serum;  for example, 200/ indicates that the titer lies between 200 and
400.

Table 6:

ANTI-HSV ANTIBODY RESPONSE IN VACCINATED CHIMPANZEES

| Vaccine | Chimp Name | Post II | | | | Post III | | | |
|---|---|---|---|---|---|---|---|---|---|
| | | 14 days | | 28 days | | 14 days | | 28 days | |
| | | Elisa Titer | Neut. Titer | Elisa Titer | Neut. Titer | Elisa Titer | Neut. Titer | Elisa Titer | Neut. Titer |
| rgp160(100µg) 3D MPL o/w | 1 | <100 | <50 | | | | | | |

ELISA TITER: corresponds to the reciprocal of the serum dilution giving an absorbance equal to 50% of the maximal absorbance value (midpointer titer)

NEUT. TITER: corresponds to the reciprocal of serum dilution giving 100% protection against the cytopathic effect of HSV2

**Claims**

1. A vaccine or pharmaceutical composition comprising gp160 or an immunological derivative thereof and 3D Monophosphoryl lipid A with a suitable carrier.

2. The composition of claim 1 wherein the gp160 protein which has been modified to provide amino acids other than lysine or arginine at positions 502 and 510 independently.

3. The composition of claim 2 wherein the amino acids of the protein at positions 502 and 510 are selected from the group histidine, threonine, serine, asparagine, aspartic acid, glutamine and glutamic acid.

4. The composition of claim 2 or claim 3 wherein the amino acids at positions 502 and 510 are glutamic acid.

5. The composition of any one of claims 1 to 4 wherein the protein is an oligomer or dimer of an HIV gp160 protein as claimed in any one of claims 2 to 4.

6. The composition of claim 5 wherein the protein has a relative molecular weight of around 640 kDa.

7. The composition of claim 1 wherein the immunological derivative is gp120.

8. The composition of any of one of the proceeding claims wherein the carrier is an oil in water emulsion.

9. The composition of claim 8 wherein the concentration of gp160 or the immunological derivative thereof is between 10 to 150 $\mu$g/ml.

10. The composition of any of claims 1 to 9 for use in medicine.

11. A method for producing a vaccine comprising gp160 or an immunological derivative thereof, 3D monophosphoryl lipid A with a pharmaceutically acceptable carrier, the method comprising mixing gp160 or immunological derivative thereof, 3D monophosphoryl lipid A and the carrier.

12. The method of claim 11 wherein the immunological derivative is gp120.

European Patent Office

**EUROPEAN SEARCH REPORT**

Application Number

EP 94 20 2409

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | INT.CONF.AIDS, 20-23 JUNE 1990 S.L.HU ET AL 'Immunisation with HIV-1 gp160 enhances HIV-specific immune responses elicited by vaccinia-HIV recombinant virus' | 1,7-12 | C07K14/16 C12N15/49 A61K39/39 A61K39/21 |
| Y | see abstract Th.A.343 --- | 2-6 | |
| Y | PROTEIN ENGINEERING, vol.2, no.3, September 1988, OXFORD pages 219 - 225 M.P.KIENY ET AL 'Improved antigenicity of the HIV env protein by cleavage site removal' * the whole document * --- | 2-6 | |
| Y | EP-A-0 335 635 (THE BOARD OF TRUSTEES OF THE LELAND STANFORD JUNIOR UNIVERSITY) 4 October 1989 * the whole document * --- | 2-6 | |
| Y | PROC. NATL. ACAD. SCI., vol.87, January 1990, USA pages 648 - 652 P.L.EARL ET AL 'Oligomeric structure of the human immunodeficiency virus type 1 envelop glycoprotein' * the whole document * --- | 5,6 | TECHNICAL FIELDS SEARCHED (Int.Cl.5) C07K A61K C12N |
| A | J.AIDS, vol.3, no.3, 1990, NEW YORK pages 200 - 205 K.N.MASIHI ET AL 'Exacerbation of human immunodeficiency virus infection in promonocytic cells by bacterial immunomodulators' see pages 200-201; page 203, column 1; page 204, column 2 - page 205; figure 3 --- -/-- | 1-12 | |

The present search report has been drawn up for all claims

| Place of search | Date of completion of the search | Examiner |
|---|---|---|
| THE HAGUE | 19 January 1995 | Groenendijk, M |

EPO FORM 1503 03.82 (P04C01)

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | CHEMICAL ABSTRACTS, vol. 107, no. 3, 20 July 1987, Columbus, Ohio, US; abstract no. 17442, M.A.TOMAI ET AL 'The adjuvant properties of a non-toxic monophosphoryl lipid A in hyporesponsive and aging mice' page 38 ;column 2 ; * abstract * & J. BIOL. RESPONSE MODIF., vol.6, no.2, 1987 page 99-107 --- | 1-12 | |
| A | J.L.BITTLE ET AL 'VACCINE BIOTECHNOLOGY' 1989 , ACADEMIC PRESS , SAN DIEGO * page 321, paragraph D * --- | 8,9 | |
| A | PROCEEDINGS OF THE NATIONAL ACADEMY OF SCIENCES OF USA., vol.83, 1986, WASHINGTON US W.G.ROBEY ET AL 'Prospect for the prevention of HIV infection;purified 120-kDa envelope glycoprotein induces neutralising antibody' * the whole document * ----- | 7,12 | TECHNICAL FIELDS SEARCHED (Int.Cl.5) |

The present search report has been drawn up for all claims

| Place of search | Date of completion of the search | Examiner |
|---|---|---|
| THE HAGUE | 19 January 1995 | Groenendijk, M |

EPO FORM 1503 03.82 (P04C01)